## Europäisches Patentamt

## European Patent Office

## Office européen des brevets

(19)

(11) Veröffentlichungsnummer: **0 121 758**
**B1**

(12) # EUROPÄISCHE PATENTSCHRIFT

(45) Veröffentlichungstag der Patentschrift:
11.06.86

(51) Int. Cl.⁴: **C 07 D 307/62**

(21) Anmeldenummer: **84102365.8**

(22) Anmeldetag: **05.03.84**

(54) Verfahren zur Herstellung von Fettsäureestern der Ascorbinsäure.

(30) Priorität: **12.03.83 DE 3308922**

(43) Veröffentlichungstag der Anmeldung:
**17.10.84 Patentblatt 84/42**

(45) Bekanntmachung des Hinweises auf die Patenterteilung:
**11.06.86 Patentblatt 86/24**

(84) Benannte Vertragsstaaten:
**CH DE FR GB LI NL**

(56) Entgegenhaltungen:
**CH - A - 418 324**
**DE - A - 2 743 526**
**DE - A - 2 854 353**
**DE - C - 639 776**

**CHEMICAL ABSTRACTS, Band 59, Nr. 2, 22. Juli 1963, Columbus, Ohio, USA P.A. POTTIER "Esters from fatty acid esters and hydroxycarboxylic acids" Zusammenfassung-Nr. 1 494 f**
**JOURNAL OF THE AMERICAN OILCHEMISTS'SOCIETY, Band 54, Nr. 7, Juli 1977 R.C. COUSINS et al. "Synthesis of 6-Fatty Acid Esters of L-Ascorbic Acid" Seiten 308-312**

(73) Patentinhaber: **BASF Aktiengesellschaft,
Carl-Bosch-Strasse 38, D-6700 Ludwigshafen (DE)**

(72) Erfinder: **Nickels, Helmut, Dr., Ginsterstrasse 15,
D-6704 Mutterstadt (DE)**
Erfinder: **Hackenberger, Alfred, Dr., Luitpoldstrasse 77,
D-6700 Ludwigshafen (DE)**

**Beschreibung**

Die vorliegende Erfindung betrifft ein verbessertes Verfahren zur Herstellung von Fettsäureestern der Ascorbinsäure.

Fettsäureester der Ascorbinsäure, wie die Ester der Palmitin-, der Myristin- und der Stearinsäure haben bekanntlich als wirksame Antioxydantien bei sehr fetthaltigen Nahrungsmitteln Bedeutung. Es hat daher nicht an Versuchen gefehlt, ein möglichst vorteilhaftes Verfahren zur Herstellung dieser Ester zu entwickeln.

So ist beispielsweise aus der US-PS 2 350 435 ein Verfahren zur Herstellung von 6-0-Fettsäureestern der Ascorbinsäure bekannt, bei dem überschüssige Ascorbinsäure in 95%iger Schwefelsäure mit höheren gesättigten aliphatischen Fettsäuren umgesetzt werden. Da die Ascorbinsäure normalerweise die teuerste Ausgangsverbindung ist und die überschüssige Ascorbinsäure nach der Umsetzung aus dem Reaktionsgemisch nicht gut wiedergewonnen werden kann, erwies sich dies Verfahren bezüglich der auf Ascorbinsäure bezogenen Ausbeuten technisch als wenig geeignet.

Zur Verbesserung der Ausbeuten an Fettsäureestern der Ascorbinsäure wurde daher gemäß der DE-OS 27 43 526 (= US 4,151,178) empfohlen, die Umsetzung in mindestens etwa 96 %iger, vorzugsweise 98 bis 99 %iger, Schwefelsäure vorzunehmen, darüber hinaus die Fettsäuren - anstelle der Ascorbinsäure in einem molaren Überschuß einzusetzen sowie bestimmte Konzentrationsbereiche der Reaktionspartner einzuhalten. Gemäß diesem Verfahren können die gewünschten Fettsäureester in Ausbeuten von bis zu 85 % der Theorie erhalten werden.

Die Fettsäureüberschüsse, die zur Erzielung der guten Ausbeuten erforderlich sind, bringen jedoch erhebliche Nachteile mit sich. Fettsäuren erschweren aufgrund ihrer Tendenz zur Emulsionsbildung die Aufarbeitung. Man kann die Ascorbinsäureester nicht einfach abfiltrieren, sondern muß sie aus dem Reaktiansgemisch extrahieren. Zu dieser extraktiven Aufarbeitung sind nur bestimmte Lösungsmittel, wie Ether, geeignet. Bezogen auf das ursprüngliche Schwefelsäurevolumen sind 20 - 30 fache Lösungsmittelmengen erforderlich. Zur Vermeidung van Emulsionen muß außerdem vorsichtig mit Kochsalzlösung gewaschen werden.

Gemäß dem Verfahren der DE-OS 2 854 353 werden trotz Verwendung äquimolarer Mengen an Ascorbinsäure und Fettsäuren hohe Ausbeuten an 6-0-Ascorbin-säureestern erhalten. Nachteilig an diesem Verfahren ist jedoch, daß als Reaktionsmedium wasserfreier Fluorwasserstoff notwendig ist.

Es war daher die Aufgabe der Erfindung, ein Verfahren zur Herstellung von Fettsäureestern der Ascorbinsäure zu entwickeln, bei dem man die gewünschten Ascorbinsäureester auf möglichst einfache Weise auch ohne Verwendung von Ascorbinsäure- bzw. Fettsäureüberschüssen und ohne die technisch problematische Verwendung von Fluorwasserstoff als Reaktionsmedium in guten Ausbeuten erhält.

Es wurde nun überraschenderweise gefunden, daß sich die beschriebenen Nachteile der bekannten Verfahren vermeiden lassen, wenn man zur Herstellung der Ascorbinsäureester die Ascorbinsäure in mehr als etwa 96%iger Schwefelsäure mit dem Methyl- oder Ethylester der entsprechenden Fettsäure anstelle der Fettsäure selbst umsetzt.

Die Umsetzung von Fettsäureestern mit Ascorbinsäure zur Herstellung von Antioxydantien ist zwar aus der BE-PS 611 648 bekannt, jedoch wird bei dem in dieser Patentschrift beschriebenen Erhitzen der genannten Ausgangsstoffe auf 90 bis 120°C nur ein unübersichtliches Produktgemisch erhalten.

Setzt man dagegen ein etwa äquimolares Gemisch von Fettsäurealkylestern und Ascorbinsäure in hochkonzentrierter Schwefelsäure um, so erhält man überraschenderweise gleich gute Ausbeuten an 6-0-Fettsäureestern der Ascorbinsäure wie bei der Verwendung von 36%igen Fettsäureüberschüssen.

Gegenstand der Erfindung ist dementsprechend ein Verfahren zur Herstellung von Fettsäureestern der Ascorbinsäure, das dadurch gekennzeichnet ist, daß man ein homogenes Gemisch aus

a) Ascorbinsäure

b) konzentrierter Schwefelsäure mit einer Konzentration von mindestens etwa 96% und

c) dem Methyl- oder Ethylester einer Fettsäure mit 12 bis 18 C-Atomen bei Temperaturen von 20 bis 50°C umsetzt.

Als Ascorbinsäure bezeichnen wir alle Isomere der Ascorbinsäure wie die L-Ascorbinsäure oder die D-Isoascorbinsäure. Bevorzugt wird das natürliche Isomere, die L-Ascorbinsäure, eingesetzt.

Als Fettsäureester verwendet man vorzugsweise die Ester von Laurinsäure, Palmitinsäure, Myristinsäure oder Stearinsäure, insbesondere deren Methylester.

Die Konzentration der Schwefelsäure sollte größer sein als 96 %. Die besten Ausbeuten werden mit etwa 100%iger Schwefelsäure erzielt.

Die Reaktion wird vorzugsweise bei Raumtemperatur durchgeführt; die Reaktionszeiten betragen im allgemeinen etwa 10 - 25 Stunden.

Die Ascorbinsäure und den Fettsäureester verwendet man mit Vorteil in etwa äquimolaren Mengen. Durch den Wegfall von überschüssigen Fettsäuren im Reaktionsgemisch vereinfacht sich dessen Aufarbeitung beträchtlich.

Zur Durchführung der Erfindungsgemäßen Umsetzung geht man mit Vorteil so vor, daß man die Ascorbinsäure bei Raumtemperatur in der konzentrierten Schwefelsäure löst, sodann den Fettsäureester zugibt, noch einige Minuten rührt und dann das Reaktionsgemisch bei Raumtemperatur stehen läßt.

Zur Aufarbeitung gießt man das Reaktionsgemisch auf Eiswasser. Hierbei kristallisiert der Ascorbinsäureester aus und kann einfach abfiltriert werden. Die gemäß den bekannten Verfahren notwendige technisch sehr aufwendige Extraktion einschließlich der destillativen Aufarbeitung der Extrakte kann entfallen. Der Filtrationsrückstand kann nach dem Trocknen aus einem geeigneten Lösungsmittel umkristallisiert werden.

In der DE-OS 27 43 526 wird neben den Fettsäureüberschüssen auch ein begrenzter Konzentrationsbereich der Reaktionspartner als wichtige Voraussetzung für gute Ausbeuten angegeben. Das Molverhältnis der Summe von Fettsäure und Ascorbinsäure zur Schwefelsäure soll zwischen 0,1 und 0,3 liegen, vorzugsweise zwischen 0,15 und 0,17. Für die wirtschaftlich besonders interessante Palmitinsäure liegt bei Raumtemperatur die obere Grenze aus Löslichkeitsgründen bei etwa 0,15. Die gleichen Autoren erwähnen in J.Am. Oil chem. Soc. 1977, 54, 308, daß bei einem Überschuß von 36 % Palmitinsäure in 99%iger Schwefelsäure (bei 20°C) die höchstmögliche Ascorbinsäurekonzentration 1,25 mol/l beträgt (entsprechend einem Molverhältnis von 0,15). Die in den Vorzugsbeispielen der genannten DE-OS angegebenen Molverhältnisse liegen z.B. für Palmitinsäure bei 0,125; das entspricht einer Ascorbinsäurekonzentration von i mol/l.

Die niederen Alkylester der Fettsäuren hingegen haben eine erheblich bessere Löslichkeit in Schwefelsäure als die Fettsäuren selbst, so daß bei der erfindungsgemäßen Umsetzung die Ascorbinsäurekonzentration im Reaktionsgemisch erhöht werden kann. Darüber hinaus zeigte sich, daß bei Ascorbinsäurekonzentrationen größer als etwa 1,3 mol/l mit äquivalenten Palmitinestermengen Ausbeuten bis zu 85 % erzielt werden können. Bei der erfindungsgemäßen Umsetzung mit Palmitinestern läßt sich die Ascorbinsäurekonzentration bis zu etwa 2 mol/l erhöhen. Hierdurch erniedrigt sich die zur Umsetzung benötigte Schwefelsäuremenge erheblich, was zur einer weiteren Vereinfachung des Aufarbeitungsschrittes führt.

Mit Hilfe des erfindungsgemäßen Verfahrens gelangt man auf technisch einfachere und dadurch billigere Weise jedoch mit gleichguten Ausbeuten zu den als Antioxydantien begehrten Fettsäureestern der Ascorbinsäure, insbesondere dem Ascorbinsäurepalmitat.

**Beispiele 1 bis 3**

Jeweils 3,5 g (0,02 mol) L-Ascorbinsäure wurden bei Raumtemperatur in 15 ml (0,28 mol) 100%iger Schwefelsäure gelöst und zu dieser Lösung 5 4 g (0,02 mol) Palmitinsäuremethylester bzw. 5,68 g (0,02 mol) Palmitinsäureethylester in flüssiger Form auf einmal addiert. Anschließend wurde das Reaktionsgemisch einige Minuten gerührt und dann für die aus der Tabelle 1 ersichtliche Zeit bei Raumtemperatur stehengelassen. Anschließend wurde das Reaktionsgemisch auf Eiswasser gegossen und filtriert. Der Filtrationsrückstand wurde mit Wasser säurefrei gewaschen und nach dem Trocknen umkristallisiert. In Tabelle 1 sind die jeweils im Reaktionsgemisch vorhandenen Konzentrationen von Ascorbinsäure und dem Palmitinsäureester, die Reaktionszeiten und die erzielten Ausbeuten angegeben.

**Tabelle 1**

| Beisp. | Ascorbinsäure [mol/l] | Palmitinsäure-ester [mol/l] | Zeit [h] | Ausbeute [%] |
|---|---|---|---|---|
| 1 | 1,32 | 1,32* | 20 | 80,2 |
| 2 | 1,32 | 1,32* | 24 | 85,1 |
| 3 | 1,32 | 1,32** | 24 | 78 |

*) Palmitinsäuremethylester
**) Palmitinsäureethylester

**Vergleichsbeispiele 1 bis 3**

Jeweils 3,5 g (0,02 mol) L-Ascorbinsäure wurden bei Raumtemperatur in 20 ml (0,37 mol) 100%iger Schwefelsäure gelöst und zu dieser Lösung 5,13 g (0,02 mol) bzw. 6,9 g (0,027 mol) Palmitinsäure addiert. Es wurde gerührt bis das Reaktionsgemisch homogen war, wozu etwa 1-2 h notwendig waren und dann das Reaktionsgemisch bei Raumtemperatur stehengelassen.

Nach Ablauf der aus Tabelle 2 ersichtlichen Reaktionszeit wurde das Reaktionsgemisch auf ca. 300 ml Eiswasser gegossen und anschließend analog OE-OS 27 43 526 extraktiv aufgearbeitet.

In Tabelle 2 sind die jeweils im Reaktionsgemisch vorhandenen Konzentrationen von Ascorbinsäure und der Palmitinsäure, die Reaktionszeiten und die erzielten Ausbeuten angegeben.

**Tabelle 2**

| Vergl. beisp. | Ascorbinsäure [mol/l] | Palmitinsäure [mol/l] | Zeit [h] | Ausbeute [%] |
|---|---|---|---|---|
| 1 | 0,99 | 0,99 | 20 | 66,3 |
| 2 | 0,99 | 0,99 | 24 | 72,3 |
| 3 | 0,99 | 1,34 | 36 | 85 |

**Beispiele 4 bis 6**

Analog den Beispielen 1 bis 3 wurde L-Ascorbinsäure in 100%iger Schwefelsäure mit äquimolaren Mengen von Laurinsäuremethylester, Myristinsäuremethylester und Stearinsäuremethylester umgesetzt. In Tabelle 3 sind die jeweils im Reaktionsgemisch vorhandenen Konzentrationen der Ascorbinsäure und der Fettsäuremethylester, die Reaktionszeiten und die erzielten Ausbeuten angegeben.

Eingesetzt wurden folgende Fettsäuremethylester:

$CH_3 - CH_2 - COOCH_3$

Laurinsäuremethylester (n = 10)

Myristinsäuremethylester (n = 12)

und Stearinsäuremethylester (n = 16)

**Tabelle 3**

| Beisp. | Ester [n] | Ascorbinsäure [mol/l] | Methylester [mol/l] | Zeit [h] | Ausbeute [%] |
|---|---|---|---|---|---|
| 4* | 10 | 1,66 | 1,66 | 24 | 90,6* |
| 5 | 12 | 1,66 | 1,66 | 24 | 75,1 |
| 6 | 16 | 1,33 | 1,33 | 30 | 70,0 |

* Zum Vergleich: laut US 4,151,178 wurden trotz Verwendung eines 36%igen Überschußes von Laurinsäure nur Ausbeuten von 86% er Theorie erhalten.

**Patentanspruch**

Verfahren zur Herstellung von Fettsäureestern der Ascorbinsäure, dadurch gekennzcichnet, daß man ein homogenes Gemisch aus

a) Ascorbinsäure,

b) konzentrierter Schwefelsäure mit einer Konzentration von mindestens 96 % und

c) dem Methyl- oder Ethylester einer Fettsäure mit 12 bis 18 C-Atomen

bei Temperaturen von 20 bis 50°C umsetzt.

**Claim**

A process for the preparation of a fatty acid ester of ascorbic acid, wherein a homogeneous mixture of

a) ascorbic acid,

b) concentrated sulfuric acid having a concentration of not less than 96%, and

c) a methyl or ethyl ester of a fatty acid of 12 to 18 carbon atoms

is reacted at from 20 to 50°C.

**Revendication**

Procédé pour la préparation d'esters d'acides gras avec l'acide ascorbique, caractérisé en ce qu'on fait réagir, à des températures de 20 à 50°C, un mélange homogène de :

a) acide ascorbique,

b) acide sulfurique concentré, d'une concentration de 96 % au moins et

c) l'ester méthylique ou éthylique d'un acide gras à 12 - 18 atomes de C.